# FASCICULE DE BREVET EUROPEEN

(11) **EP 3 248 624 B1**
(45) Date de publication et mention de la délivrance du brevet: **22.09.2021**
(21) Numéro de dépôt: 17169826.9
(22) Date de dépôt: 13.07.2011
(51) Int. Cl.: A61M 1/00, A61M 27/00, A61B 17/12, A61B 17/42, A61B 17/30, A61B 17/00

(54) **DISPOSITIF POUR LE CONTRÔLE D'UN ÉCOULEMENT SANGUIN SE PRODUISANT DANS UNE ZONE HÉMORRAGIQUE**
VORRICHTUNG ZUR KONTROLLE EINES BLUTFLUSSES IN EINER HÄMORRHAGISCHEN ZONE
DEVICE FOR MONITORING A BLOOD FLOW OCCURRING IN A HAEMORRHAGIC REGION

(30) Priorité: 13.07.2010 FR 1055736
(43) Date de publication de la demande: 29.11.2017
(62) Demande divisionnaire de: 11754694.5
(73) Titulaire: Hemosquid, 38400 Saint Martin d'Heres (FR)
(72) Inventeur: BLIN, Dominique, 38700 La Tronche (FR)
(74) Mandataire: Regimbeau

(56) Documents cités:
- DE-U1-202009 011 664
- US-A- 5 034 006
- US-A1- 2007 282 309

## Description

### Domaine technique

La présente divulgation concerne un dispositif pour le contrôle d'un écoulement sanguin se produisant dans une zone hémorragique, notamment dans une zone ponctuelle (dont le plus grand diamètre est inférieur à environ 10 mm) du corps humain ou plus généralement animal.

Un tel dispositif permet par exemple à un utilisateur de contrôler et d'arrêter un écoulement sanguin important se produisant :
- dans une zone de tissu biologique fortement vascularisée, appartenant notamment à une cavité naturelle interne du corps d'un patient et qui par conséquent présente un accès difficile, cet écoulement sanguin créant dans cette zone une forte hémorragie ;
- après une blessure vasculaire, que ce soit au cours d'une intervention chirurgicale ou après un traumatisme. Cet écoulement pouvant se trouver dans une région profonde, difficile d'accès, dans une cavité naturelle comme l'estomac ou l'utérus. Ce saignement peux être impossible à accéder directement comme en vidéo-chirurgie. Le dispositif selon la divulgation vient s'ajouter aux techniques conventionnelles d'hémostase qui peuvent être inefficaces dans de telles circonstances.

### Etat de la technique antérieure

On connaît déjà (voir par exemple la demande de brevet WO2009101348) un dispositif d'hémostase pour contrôler et arrêter un écoulement sanguin, en réalisant, au droit d'une plaie ou contre une zone de tissu mise à nu et directement accessible, un drainage aspiratif qui provoque une évacuation des particules mortes infectées ou non et une certaine migration des tissus favorisant cette hémostase.

US 2007 282309 décrit un ensemble de drainage de plaie pour transporter un fluide à partir d'un organe creux en appliquant une pression négative provenant d'une source extérieure à l'organe creux à travers l'ensemble de drainage de plaie qui est placé directement à l'intérieur de l'organe creux.

Un premier problème technique se posant à la vue de l'état de la technique est le bon maintien d'un tel dispositif contre cette plaie ou ce tissu.

Un deuxième problème technique se posant à la vue de l'état de la technique est le maintien rapproché deux parois opposées d'un organe « creux » comme par exemple de l'utérus dans le cas des hémorragies qui peuvent se produire à l'occasion d'un accouchement lors de la délivrance d'une patiente, dans la paroi interne de son utérus.

L'objectif de l'invention est de résoudre ce premier ou deuxième problème.

### Exposé de l'invention

La présente invention est définie dans les revendications annexées.

Le premier problème est résolu avec un dispositif pour le contrôle d'un écoulement sanguin se produisant dans une zone hémorragique d'un tissu biologique, comprenant une plaque souple, cette plaque étant agencée pour être disposée en regard de cette zone, ladite plaque souple comprenant :
- des moyens d'appui périphériques sensiblement étanches, propres à s'appliquer sur le tissu biologique en entourant la zone hémorragique,
- une paroi de fond, cette paroi de fond délimitant avec les moyens d'appui, en regard de la zone, un espace creux, et
- des moyens de raccordement agencés pour raccorder l'espace creux, extérieurement à la plaque souple, à une source d'aspiration externe pour créer dans l'espace creux un vide pour aspirer et appliquer étroitement la surface du tissu contre les moyens d'appui périphériques,
caractérisé en ce qu'il comprend en outre un plot creux disposé entre la zone et la paroi de fond et évidé en son centre du côté de la zone selon un axe sensiblement perpendiculaire à la paroi de fond, et agencé pour être en contact avec la zone lorsque l'on crée le vide dans l'espace.

Le plot creux peut être agencé pour, lorsque son côté évidé est en contact avec la zone, être isolé du vide crée dans l'espace.

Le plot creux peut ne pas comprendre de trou reliant son intérieur évidé à l'espace.

Le plot creux peut avoir une forme cylindrique d'axe de symétrie sensiblement perpendiculaire à la paroi de fond.

Le plot creux peut être agencé pour être rempli d'un produit comme de la colle hémostatique ou un produit hémostatique.

La paroi de fond peut être agencée pour être percée par une seringue pour injecter un produit comme de la colle ou produit hémostatique dans le plot creux sans traverser les moyens de raccordement entre l'espace creux et les moyens d'aspiration.

Les moyens de raccordement peuvent être agencés pour raccorder l'espace creux sans logement collecteur intermédiaire ménagé dans l'épaisseur de la paroi de fond.

Le plot creux peut être centré sur la paroi de fond.

Le plot creux peut être agencé pour accueillir du côté de la zone une couronne évidée et entourant le plot creux de sorte que la jonction entre le plot creux et la couronne soit hermétique.

Le dispositif selon la divulgation peut comprendre une couronne évidée et entourant le plot creux du côté de la zone de sorte que la jonction entre le plot creux et la couronne soit hermétique.

La couronne peut avoir une forme cylindrique.

La couronne peut avoir une forme évasée en direction de la zone.

Le dispositif selon la divulgation peut comprendre une jupe disposée entre les moyens d'appui périphériques et le plot creux en entourant le plot creux, et agencée pour être en contact avec la zone lorsque l'on crée le vide dans l'espace. La jupe peut être centrée sur la paroi de fond. La jupe peut être munie de dents dirigées vers la zone et agencées pour être en contact avec la zone lorsque l'on crée le vide dans l'espace.

Les moyens d'appui périphériques peuvent être agencés pour accueillir une pièce additionnelle permettant de modifier la surface de plaque ou d'en modifier le profil.

La paroi de fond peut être étanche et imperméable.

Le dispositif selon la divulgation peut intégrer une caméra pour aider à positionner la plaque sur zone.

Suivant l'invention, le deuxième problème est résolu avec un dispositif hémostatique pour le contrôle d'un écoulement sanguin se produisant dans une zone hémorragique située dans un organe creux, comprenant :
- une plaque souple munie de deux faces opposées, ladite plaque comprenant un volume interne vide entre ses deux faces, et
- des moyens de raccordement agencés pour raccorder le volume interne à des moyens d'aspiration extérieur audit dispositif de manière à créer une dépression dans ce volume interne,
chacune des faces étant munie de trous faisant communiquer, via ces faces, le volume interne et l'extérieur de la plaque de telle sorte que lorsque ladite dépression est créée dans le volume interne, l'aspiration peut être communiquée simultanément à deux parois opposées de l'organe creux de manière à les maintenir rapprochées.

Chacune des faces de la plaque peut être munie, vers l'extérieur du dispositif, de motifs en reliefs.

Les motifs peuvent être répartis sur les faces de manière régulière, avec une densité surfacique sensiblement constante.

Suivant encore un autre aspect de la divulgation, il est proposé une méthode pour le contrôle d'un écoulement sanguin se produisant dans un espace clôt biologique, dans laquelle on dispose le dispositif selon la divulgation sur la zone hémorragique.

### Description des figures et modes de réalisation

D'autres avantages et particularités de la divulgation apparaîtront à la lecture de la description détaillée de mises en œuvre et de modes de réalisation nullement limitatifs, et des dessins annexés suivants :
- la figure 1 est une vue de coupe de profil d'un premier mode de réalisation de dispositif selon la divulgation appelé « petite plaque »,
- la figure 2 est une vue de dessous du premier mode de réalisation de dispositif selon la divulgation de la figure 1,
- la figure 3 est une vue de coupe de profil d'un deuxième mode de réalisation de dispositif selon la divulgation appelé « grande plaque »,
- la figure 4 est une vue de dessous du deuxième mode de réalisation de dispositif selon la divulgation de la figure 3,
- la figure 5 est une vue de coupe de profil d'un plot creux du premier ou deuxième mode de réalisation de dispositif selon la divulgation,
- la figure 6 est une vue de coupe de profil d'un premier mode de réalisation de couronne selon la divulgation pour entourer le plot creux du premier ou deuxième mode de réalisation de dispositif selon la divulgation,
- la figure 7 est une vue de coupe de profil d'un deuxième mode de réalisation de couronne selon la divulgation pour entourer le plot creux du premier ou deuxième mode de réalisation de dispositif selon la divulgation,
- la figure 8 est une vue de coupe de profil d'une variante de dispositif selon la divulgation,
- chacune des figures 9 à 11 est une vue de coupe de profil d'une pièce additionnelle pour le premier ou deuxième mode de réalisation de dispositif selon la divulgation,
- la figure 12 est une vue de dessous d'un mode de réalisation de dispositif selon la divulgation muni d'une « jupe »,
- la figure 13 est une vue de coupe de profil de ce mode de réalisation de dispositif selon la divulgation muni d'une « jupe »,
- la figure 14 est une vue de coupe de profil d'un mode de réalisation de dispositif selon l'invention appelé « plaque pour organes creux »,
- la figure 15 est une vue de dessus de la « plaque pour organes creux »,
- la figure 16 est une vue de coupe de face de la « plaque pour organes creux »,
- la figure 17 est une vue de coupe de profil d'un autre mode de réalisation de dispositif selon la divulgation appelé dispositif « à aspiration centrale »,
- la figure 18 est une vue de dessous du plot creux 11 du mode de réalisation « à aspiration centrale »,
- la figure 19 est une vue de coupe de profil du plot creux 11 du mode de réalisation « à aspiration centrale », selon la section A-A de la figure 18,
- la figure 20 est une vue de profil du plot creux 11 du mode de réalisation « à aspiration centrale », et
- la figure 21 est une vue de coupe de profil d'une variante de pièce additionnelle 20 pour l'un quelconque des modes de réalisation de dispositif selon la divulgation.

De manière générale, les dimensions indiquées sur les figures sont en millimètres.

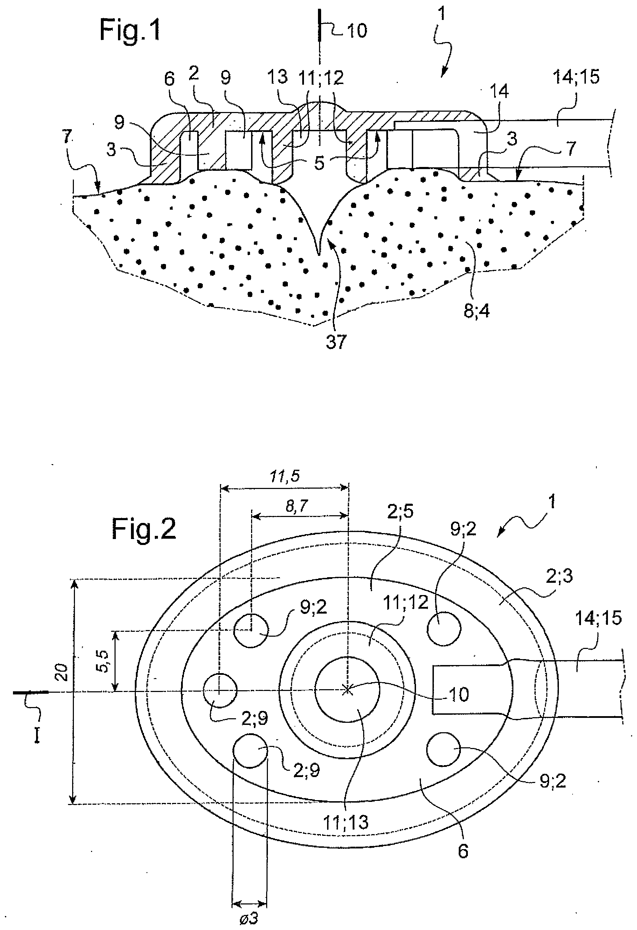

On va tout d'abord décrire, en référence aux figures 1 et 2, un premier mode de réalisation de dispositif « A1 » (référencé 1 sur les figures) selon la divulgation appelé « petite plaque ».

La figure 1 est une vue de coupe de profil selon l'axe I de la figure 2.

Le dispositif 1, pour le contrôle d'un écoulement sanguin se produisant dans une zone hémorragique 4 d'un tissu biologique 8, comprend une plaque souple 2 plate.

Vue de dessous ou de dessus, la plaque a une forme ronde ou ovale. La plaque souple 2 est réalisée en caoutchouc naturel ou synthétique ou en matière plastique du genre silicone.

La plaque souple 2 comprend :
- des moyens d'appui périphériques 3 sensiblement étanches,
- une paroi de fond 5,
- des moyens de raccordement 14 à une source d'aspiration externe au dispositif (non représentée),
- un plot creux 11.

La plaque 2 est agencée pour être disposée en regard et s'étendre parallèlement à de cette zone hémorragique 4, de sorte que :
- les moyens d'appui périphériques 3 sensiblement étanches sont appliqués sur le tissu biologique 8 en entourant la zone hémorragique 4,
- la paroi de fond 5 délimite avec les moyens d'appui 3, en regard de la zone 4, un espace creux 6,
- les moyens de raccordement 14 sont agencés pour raccorder l'espace creux 6, extérieurement à la plaque souple 2, à la source d'aspiration externe (non représentée) pour créer dans l'espace creux 6 un vide pour aspirer et appliquer étroitement la surface 7 du tissu 8 contre les moyens d'appui périphériques 3, et
- le plot creux 11 est disposé entre la zone 4 et la paroi de fond 5 et évidé en son centre du côté de la zone 4 selon un axe 10 sensiblement perpendiculaire à la paroi de fond, et est agencé pour être en contact avec la zone 4 lorsque l'on crée le vide dans l'espace 6.

Les moyens de raccordement 14 comprennent un tube 15 (ou conduit de liaison) débouchant à l'intérieur de l'espace creux 6.

Comme illustré sur les figures 1 et 2, le plot creux 11 a une forme cylindrique d'axe de symétrie 10 sensiblement perpendiculaire à la paroi de fond 5.

En outre, le plot creux 11 est centré sur la paroi de fond 5.

La paroi de fond 5 est étanche et imperméable, tout comme les moyens d'appui périphériques 3. Par imperméable, on entend qu'elle isole et ne se laisse pas se propager à travers elle un liquide ou une différence de pression.

Les moyens d'appui périphériques 3 de la plaque souple sont constitués par une bordure flexible entourant la paroi de fond 5 de cette plaque et solidarisée de celle-ci.

Le dispositif 1 comprend en outre des moyens intercalaires discontinus 9 agencés pour rigidifier la plaque souple 2, ces moyens intercalaires étant disposés entre cette zone 4 et la paroi de fond 5.

Les moyens intercalaires discontinus 9 sont destinés en outre à faciliter la propagation du vide dans l'espace creux 6 et à limiter le glissement du dispositif selon la divulgation sur la surface 7.

Les moyens intercalaires discontinus 9 comprennent des plots 9 répartis sur la paroi de fond. Les moyens intercalaires sont constitués par une pluralité de plots d'entretoisement 9, de préférence identiques, équidistants et régulièrement répartis dans l'étendue de la plaque souple 2, aptes à venir en contact par une de leurs extrémités avec la zone hémorragique, ces plots 9 étant solidaires avec la paroi de fond 5 par leurs extrémités opposées. Les plots 9 présentent un profil cylindrique plein, à section circulaire. Les moyens intercalaires 9 sont réalisés dans la même matière que la plaque, à savoir en caoutchouc naturel ou synthétique ou en matière plastique du genre silicone.

Ainsi, la plaque 2 du dispositif 1 forme une ventouse plate de recouvrement étanche, apte à entourer et à s'appliquer étroitement contre la plaie 37 ou la zone 4 de tissu 8 à traiter, siège d'une hémorragie ou d'un écoulement sanguin à contrôler, cette ventouse venant en contact avec la périphérie de la zone 4 de tissu 8 et délimitant un volume interne 6 relié de façon étanche par un cathéter ou un tube de liaison similaire 15 à un organe extérieur d'aspiration et de mise sous vide, créant dans ce volume 6, entre la ventouse et la zone 4 de tissu 8, une dépression de valeur déterminée.

Grâce à ces dispositions, la plaie 37 se referme par migration des tissus sous l'effet de la dépression créée, les organes d'appui interne 9, 11 maintenant le tissu 8 sensiblement en place.

Le plot creux 11 comprend une paroi circonférentielle 12 entourant un intérieur creux 13. La paroi 12 est imperméable. Le plot creux 11 est agencé pour que, lorsque son côté évidé est en contact avec la zone 4, sa paroi 12 entoure la plaie 37 de sorte que la plaie 37 soit disposée en regard de l'intérieur creux 13.

Le plot creux 11 est agencé pour que, lorsque son côté évidé est en contact avec la zone 4, son intérieur creux 13 est isolé du vide crée dans l'espace 6. En particulier, le plot creux 11 ne comprend pas dans sa paroi 12 de trou reliant son intérieur 13 évidé à l'espace 6.

Le plot creux 11 est agencé pour être rempli de colle ou de produit hémostatique, plus exactement l'intérieur creux 13 est agencé pour être rempli de colle ou de produit hémostatique

La paroi de fond 5 est agencée pour être percée par une seringue (par exemple selon l'axe 10, de l'extérieur du dispositif 1 vers l'intérieur creux 13) pour injecter de la colle ou du produit hémostatique dans l'intérieur creux 13 du plot 11 sans traverser les moyens de raccordement 14 entre l'espace creux 6 et les moyens d'aspiration. En particulier, les moyens de raccordement 14 sont agencés pour raccorder l'espace creux 6 directement au conduit de liaison 15 sans logement collecteur intermédiaire ménagé dans l'épaisseur de la paroi de fond 5.

Ainsi, on peut insérer de la colle ou un produit hémostatique dans l'intérieur creux 13 pour assurer un bon maintien du dispositif sur le tissu 8, en particulier un bon maintien du plot 11 sur la plaie 37, sans risquer de percer les moyens de raccordement 14 vers les moyens d'aspiration créant le vide, et sans risquer que la colle ou le produit hémostatique ne soit aspirée dans l'espace 6 puis vers les moyens d'aspiration.

Dans une autre variante, la colle ou le produit hémostatique est insérée au préalable dans l'espace creux 13 avant que la plaque 2 ne soit appliquée sur la plaie 37. Ainsi, on peut insérer de la colle ou le produit hémostatique dans l'intérieur creux 13 pour assurer un bon maintien du dispositif sur le tissu 8, en particulier un bon maintien du plot 11 sur la plaie 37, sans risquer que la colle hémostatique ne soit aspirée dans l'espace 6 puis vers les moyens d'aspiration.

On note qu'un autre produit que de la colle peut être inséré dans l'intérieur creux, par exemple un produit collant, antiseptique, cicatrisant, et/ou antibiotique

Le dispositif 1 peut par exemple être utilisé :
- pour des plaies difficiles d'accès, dans des cavités dont l'accès difficile peut rendre aléatoire le contrôle efficace de cette hémorragie par les moyens traditionnels, par exemple sur la paroi interne d'une cavité naturelle telle que la paroi interne de l'œsophage, du tube digestif au droit notamment de l'estomac entre le cardia et le duodénum, voire dans la paroi de ce dernier, du péritoine et de l'intestin et en particulier du colon,
- dans le cas de lésion ou de plaie ouverte se produisant sur un organe dont le tissu est particulièrement fragile, tel que le cœur, le foie ou la rate, où le contrôle de l'hémorragie nécessite principalement, non pas d'aspirer le sang qui s'écoule de la plaie, mais plus immédiatement à rapprocher énergiquement les lèvres de la plaie en les maintenant appliquées l'une sur l'autre par un effet de fixation consécutif à la mise en place du dispositif, cet effet refermant la lésion jusqu'à ce que l'hémorragie soit convenablement stoppée, ou à contenir le saignement dans un espace clôt,
- dans le cas de surfaces dites cruentées, créées entre deux parois ou membranes biologiques normalement en contact étroit mais écartées accidentellement l'une de l'autre par clivage, comme entre la plèvre et la paroi pulmonaire, et entre lesquelles se produit un saignement ou une hémorragie diffuse, à contrôler et arrêter rapidement.

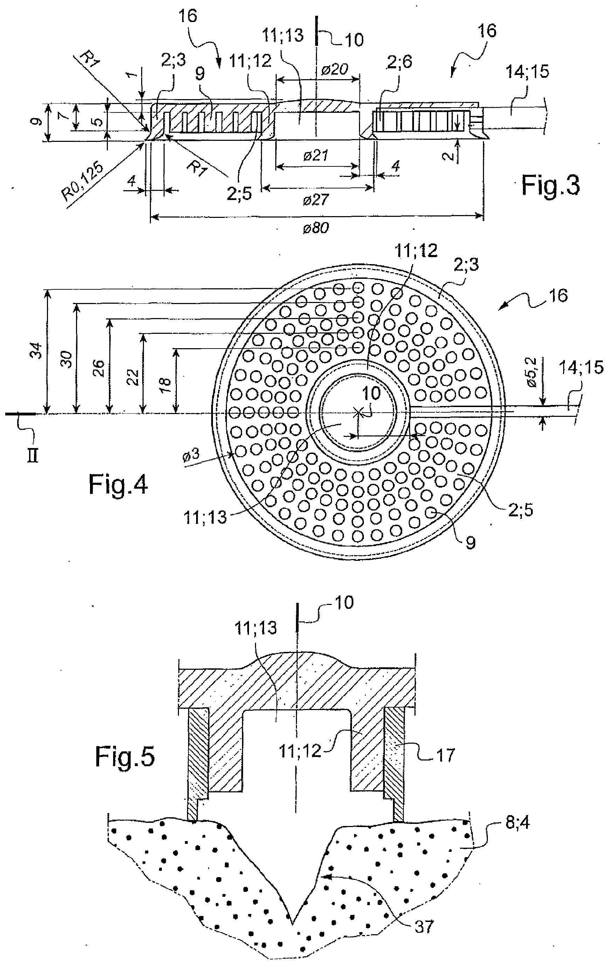

On va maintenant décrire, en référence aux figures 3 et 4, un deuxième mode de réalisation de dispositif « A2 » (référencé 16 sur les figures) selon la divulgation appelé « grande plaque », uniquement pour ses différences par rapport au premier mode de réalisation.

La figure 3 est une vue de coupe de profil selon l'axe II de la figure 4.

Ce deuxième mode de réalisation est identique au premier mode, mis à part qu'il est de plus grandes dimensions et qu'il comprend un nombre plus élevés de plots 9.

On va maintenant décrire, en référence aux figures 5 à 7, un plot creux 11 du premier ou deuxième mode de réalisation de dispositif selon la divulgation, et différents modes de réalisation de couronnes associées à ce plot creux 11.

Dans les premier et deuxième modes de réalisation de dispositif selon la divulgation, le plot creux 11 est agencé pour accueillir du côté de la zone 4 (c'est-à-dire de son côté évidé) une couronne amovible 17 évidée et entourant le plot creux 11 de sorte que la jonction entre le plot creux 11 et la couronne 17 soit hermétique.

Le dispositif 1, 16 comprend donc typiquement une couronne évidée et entourant le plot creux 11 du côté de la zone 4 de sorte que la jonction entre le plot creux 11 et la couronne soit hermétique.

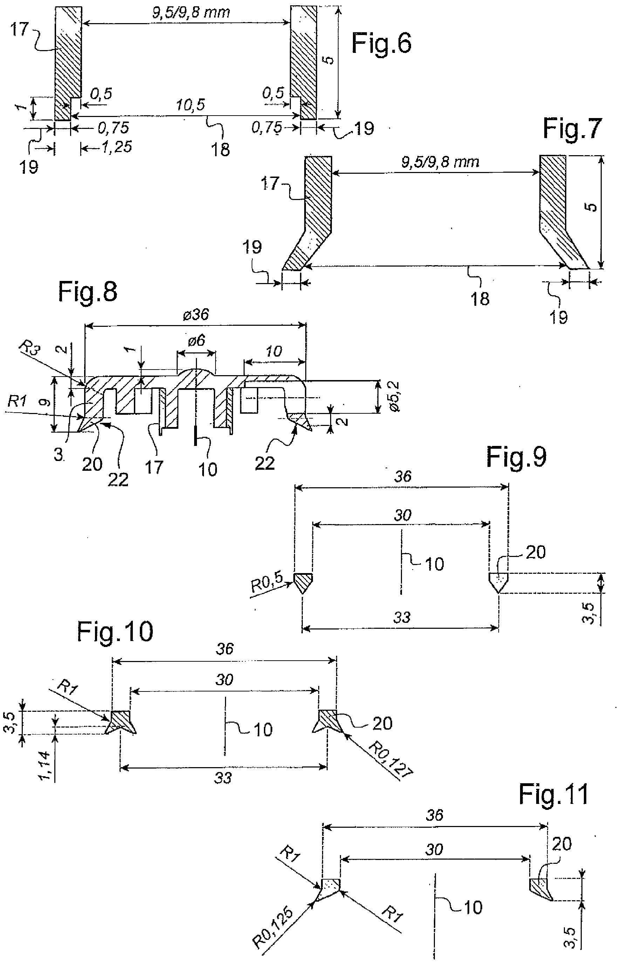

La couronne 17 a :
- une forme cylindrique, comme représentée sur la figure 6, ou
- une forme cylindrique du côté de la paroi de fond 5 et évasée en direction de la zone 4, comme représentée sur la figure 7.

La couronne a une symétrie de révolution autour de l'axe 10.

Ainsi, on peut modifier :
- le diamètre 18 de l'intérieur creux 13 du côté de la zone 4, et/ou
- la surface 19 de contact entre la couronne 17 et le tissu 8, en fonction du modèle de couronne 17 sélectionnée pour entourer le plot 11.

La couronne 17 améliore le maintien et la fermeture de la plaie.

Le plot creux peut être muni de dents microstructurées.

La couronne 17 est munie de dents microstructurées.

On va maintenant décrire, en référence aux figures 8 à 11, une pièce additionnelle dans une variante du premier ou deuxième mode de réalisation de dispositif selon la divulgation.

Dans cette variante du premier ou deuxième mode de réalisation de dispositif selon la divulgation, les moyens d'appui périphériques 3 sont agencés pour accueillir une pièce additionnelle 20 permettant de modifier la surface de plaque ou d'en modifier le profil.

La pièce additionnelle est fixée sur les moyens d'appui périphériques par collage, clipsage ou par adhésion de surfaces comme par exemple de deux surfaces lisses de caoutchouc pouvant se coller et se décoller un grand nombre de fois.

Ainsi, on peut modifier et adapter sur mesure les propriétés d'adhésion et de maintien du dispositif 1, 16 au tissu 8.

La pièce additionnelle 20 est munie de dents 22 microstructurées dirigées vers le tissu 8 et agencées pour être en contact avec le tissu 8 lorsque l'on crée le vide dans l'espace 6. La pièce additionnelle 20 assure un meilleur maintien du dispositif sur le tissu 8, par accrochage sur le tissu 8 des microstructures 22 en forme de dent.

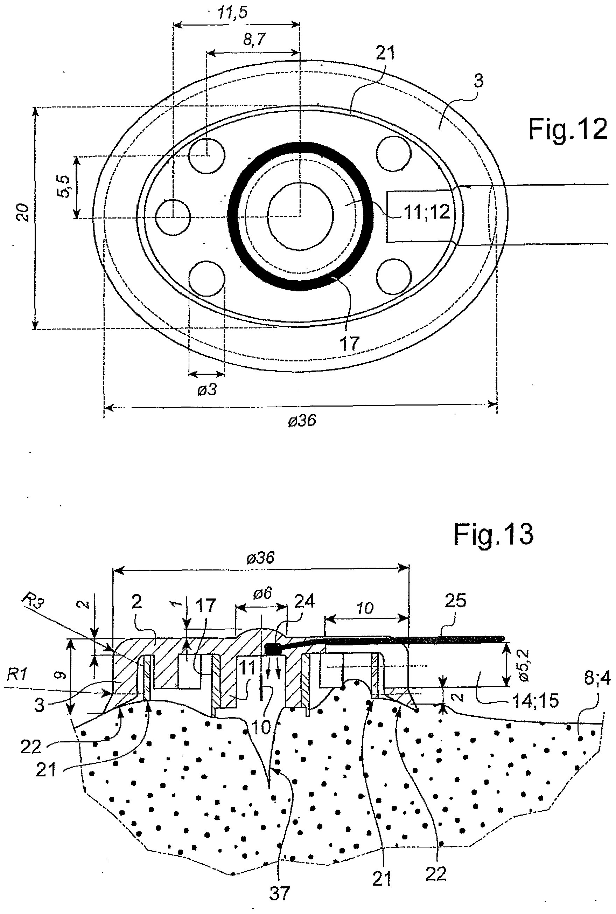

On va maintenant décrire, en référence aux figures 12 et 13, une jupe pour l'une quelconque des variantes de mode de réalisation de dispositif selon la divulgation venant d'être décrit.

L'une quelconque des variantes de mode de réalisation de dispositif selon la divulgation peut comprendre en outre, comme représenté sur les figures 12 et 13, une jupe 21 amovible disposée entre les moyens d'appui périphériques 3 et le plot creux 11 en entourant le plot creux 11, et agencée pour être en contact avec le tissu 8 lorsque l'on crée le vide dans l'espace 6.

La jupe 21 est centrée sur la paroi de fond 5.

La jupe assure un meilleur maintien du dispositif sur le tissu 8.

En outre, le dispositif 1, 16 peut intégrer une caméra 24 pour aider le praticien à positionner la plaque 2 sur la zone 4 et centrer la plaque 2 par rapport à la plaie 37.

La caméra est agencée pour visualiser le tissu via l'intérieur creux 23 lorsque le côté évidé du plot 11 est en contact avec la zone 4.

Un câble 25 relie la caméra 24 à des moyens distants (non représentés) pour recevoir des données visuelles de la caméra.

La caméra est alimentée localement (pile, batterie) ou via le câble 25.

Le câble part de la caméra 24 vers les moyens de raccordement 14 puis suit le parcours du conduit 15.

La caméra est décentrée par rapport à l'axe central 10 du plot 11, pour permettre à une seringue de percer la plaque 2 pour injecter un produit dans l'intérieur creux 13 sans risquer d'endommager la caméra 24 ou le câble 25. De façon avantageuse pour ne pas avoir de doutes sur la position de la caméra 24 et du câble, la caméra est décalée vers les moyens de raccordement 14.

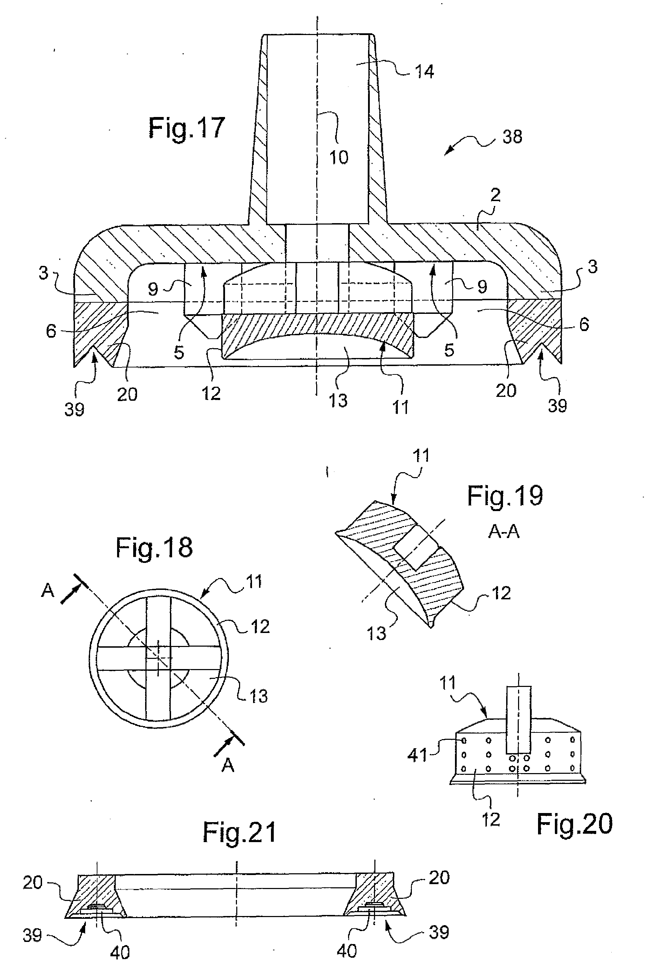

On va maintenant décrire, en référence aux figures 17 à 20, un troisième mode de réalisation de dispositif « A3 » (référencé 38 sur les figures) selon la divulgation appelé dispositif « à aspiration centrale », uniquement pour ses différences par rapport au premier mode de réalisation.

Dans ce mode de réalisation 38, les plots 9 sont des plots antidérapants.

En outre, dans ce mode de réalisation 38, les moyens de raccordement 14 débouchent directement à l'intérieur du plot creux 11, dans la base du plot creux.

Plus exactement, les moyens de raccordement 14 débouchent à l'intérieur du plot creux 11 par le dessus du plot creux du côté de la paroi de fond 5.

Le fait d'avoir une aspiration centrale, via le plot creux 11, plutôt que latérale directement dans l'espace 6 :
- évite le blocage de l'aspiration du tissu 8, et
- permet de rendre plus homogène l'aspiration.

On remarque que l'intérieur creux 13 a une forme intérieure concave.

Dans ce mode de réalisation 38, le plot creux 11 a une double fonction :
- fonction de répartition homogène de l'aspiration, et
- fonction de rigidifier le dispositif en servant de « pilier ».

Dans ce mode de réalisation 38, la paroi 12 est trouée (trous 41) pour permettre à l'aspiration d'être transmise entre l'intérieur 13 du plot 11 et l'espace 6.

Les moyens de raccordement 14 débouchent à l'intérieur du plot creux 11 avec un trou qui prend une forme de croix (illustrée sur la figure 18) de manière à améliorer la dépression et son homogénéisation.

Comme pour les modes de réalisation précédemment décrits, le mode de réalisation 38 peut être muni :
- de la couronne 17 ou bague rigide métallique (non illustrée), qui sera alors trouée pour permettre à l'aspiration d'être transmise entre l'intérieur 13 du plot 11 et l'espace 6, et/ou
- de la pièce additionnelle 20, et/ou
- de la jupe 21 (non illustrée).

Dans une variante préférentielle de l'un quelconque des modes de réalisation précédemment décrits, le dispositif selon la divulgation comprend une partie en silicone propre à s'appliquer sur le tissu biologique 8 et de dureté différente et inférieure à la dureté de la paroi de fond 5.

On sait qu'il existe les échelles Shore A, B, C, D, 0, et 00.

Dans ce document, toutes les valeurs en Shore sont données par défaut dans l'échelle Shore A, sauf spécification contraire.

La fonction de la paroi de fond 5 est d'assurer une rigidité au dispositif suffisante pour résister à la dépression due à l'aspiration. La paroi de fond a une dureté comprise entre 35 et 85 Shore A, de préférence sensiblement égale à 50 Shore A.

La fonction de la partie propre à s'appliquer sur le tissu biologique est de s'adapter à la forme de la plaie ou du tissu 8. Cette partie propre à s'appliquer sur le tissu biologique et de dureté différente à la dureté de la paroi de fond a une dureté comprise entre 0 et 50 Shore A, de préférence sensiblement égale à 35 Shore A.

La partie propre à s'appliquer sur le tissu biologique 8 et de dureté différente à la dureté de la paroi de fond comprend :
- les moyens d'appui périphériques 3 en silicone, et/ou
- la pièce additionnelle 20 en silicone, si le dispositif selon la divulgation est équipé d'une telle pièce additionnelle.

En référence à la figure 21, si le dispositif selon la divulgation est équipé de la pièce additionnelle 20, la pièce additionnelle 20 est équipée d'un joint 40 intermédiaire en silicone de dureté inférieure à la dureté de la pièce additionnelle 20 et disposé de manière à être situé entre la pièce additionnelle 20 et le tissu biologique. Cela permet de s'adapter encore mieux à la forme de la plaie ou du tissu 8. La pièce additionnelle 20 est munie d'une fente 39 en forme de boucle fermée et agencée pour accueillir le joint 40. Le joint 40 a une dureté comprise entre 0 et 10 Shore 00 (i.e. selon l'échelle Shore 00), de préférence sensiblement égale à 5 Shore 00.

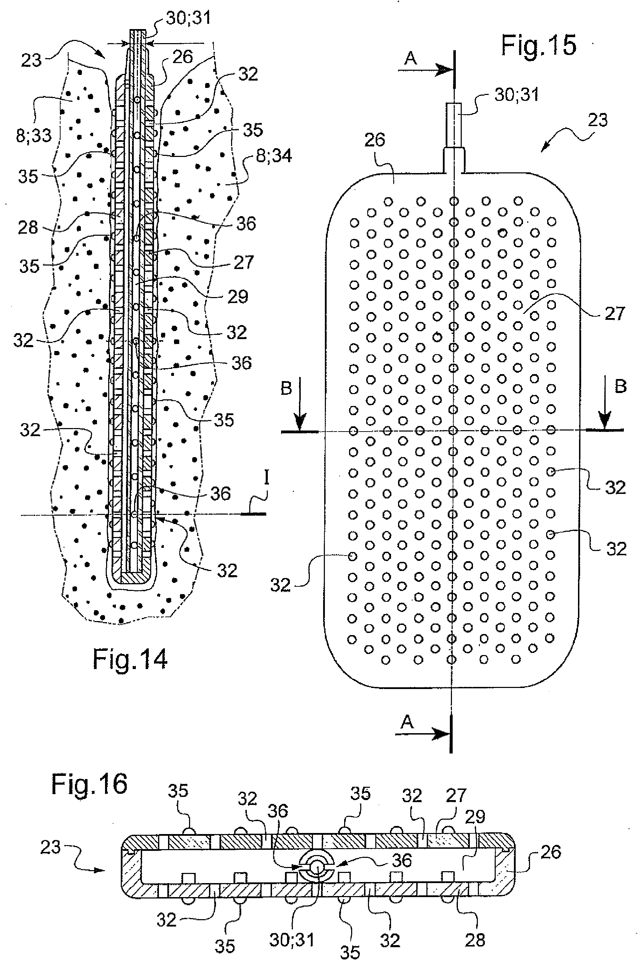

On va maintenant décrire, en référence aux figures 14 à 16, un mode de réalisation de l'invention « B » (référencé 23 sur les figures) pouvant faire l'objet d'une demande divisionnaire, toujours dans le domaine des plaques pour hémostase.

La figure 14 est une vue de coupe de profil selon l'axe A-A de la figure 15.

La figure 16 est une vue de coupe de profil selon l'axe I de la figure 14.

La figure 3 est une vue de coupe de profil selon l'axe II de la figure 4.

Le dispositif 23 comprend :
- une plaque souple 26 plate munie de deux faces 27, 28 opposées sensiblement parallèles, ladite plaque comprenant un volume interne 29 entre ses deux faces, et
- des moyens de raccordement 30 agencés pour raccorder le volume interne 29 à des moyens d'aspiration (non représentés) extérieur audit dispositif de manière à créer une dépression dans ce volume interne 29 , les dits moyens de raccordement comprenant typiquement un tube de liaison 31
chacune des faces 27, 28 étant munie de trous 32 faisant communiquer via ces faces 27, 28 le volume interne 29 et l'extérieur de la plaque.

Ainsi, le dispositif peut communiquer l'aspiration au tissu 8 simultanément via les deux faces opposées 27, 28, et permet ainsi de maintenir rapprochées deux parois opposées 33, 34 d'un organe « creux » comme par exemple de l'estomac ou l'œsophage, ou de manière préférentielle de l'utérus dans le cas des hémorragies qui peuvent se produire à l'occasion d'un accouchement lors de la délivrance d'une patiente, dans la paroi interne de son utérus.

En outre, chacune des faces 27, 28 de la plaque 23 est munie, vers l'extérieur du dispositif 23, de motifs en reliefs 35 en forme de bosse.

Les motifs 25 sont répartis sur les faces 27, 28 de manière régulière, avec une densité surfacique sensiblement constante. Pour des raisons de simplification, ces motifs ne sont pas illustrés sur la figure 15.

Le rôle de ces motifs 35 est d'améliorer l'étanchéité du dispositif 23 aux divers endroits du saignement.

Le tube 31 se prolonge à l'intérieur du volume 29, sur toute la longueur du volume 29.

Ce tube est muni, sur toute sa longueur à l'intérieur de l'espace 29, de paires de trous latéraux 36 qui font communiquer l'intérieur du tube 31 au volume 29, de sorte que la dépression soit bien uniforme à l'intérieur de l'espace 29.

Le dispositif « B » (référencé 23 sur les figures) peut par exemple être utilisé :
- dans le cas des hémorragies ou de saignements étouffés qui peuvent se produire à l'occasion d'un accouchement lors de la délivrance d'une patiente, dans la paroi interne de son utérus, lorsque celui-ci est relativement atone, surtout dans la région de l'insertion placentaire qui n'est plus exactement localisable de manière précise dans la période qui suit cette délivrance et l'expulsion du placenta.

Bien sûr, l'invention n'est pas limitée aux exemples qui viennent d'être décrits et de nombreux aménagements peuvent être apportés à ces exemples sans sortir du cadre de l'invention.

## Revendications

1. Dispositif (23) hémostatique pour le contrôle d'un écoulement sanguin se produisant dans une zone hémorragique (4) située dans un organe creux, comprenant :
- une plaque souple (26) plate munie de deux faces (27, 28) opposées, sensiblement parallèles, ladite plaque comprenant un volume interne (29) vide entre ses deux faces, et
- des moyens de raccordement (30) agencés pour raccorder le volume interne (29) à des moyens d'aspiration extérieurs audit dispositif, de manière à créer une dépression dans ce volume interne (29),
chacune des faces (27, 28) étant munie de trous (32) faisant communiquer, via ces faces (27, 28), le volume interne (29) et l'extérieur de la plaque de telle sorte que lorsque ladite dépression est créée dans le volume interne, l'aspiration peut être communiquée simultanément à deux parois opposées de l'organe creux de manière à les maintenir rapprochées.

2. Dispositif selon la revendication 1, **caractérisé en ce que** chacune des faces (27, 28) de la plaque est munie, vers l'extérieur du dispositif (23), de motifs en reliefs (35) en forme de bosse.

3. Dispositif selon la revendication 2, **caractérisé en ce que** les motifs (35) sont répartis sur les faces (27, 28) de manière régulière, avec une densité surfacique sensiblement constante.

4. Dispositif selon l'une quelconque des revendications précédentes, **caractérisé en ce que** lesdits moyens de raccordement comprennent un tube de liaison (31), et **en ce que** le tube (31) se prolonge à l'intérieur du volume (29), sur toute la longueur du volume (29).

5. Dispositif selon la revendication 4, **caractérisé en ce que** le tube (31) est muni, sur toute sa longueur qui se prolonge à l'intérieur du volume, de paires de trous latéraux (36) qui font communiquer l'intérieur du tube (31) au volume interne (29), de sorte que la dépression soit bien uniforme dans le volume interne (29).

6. Dispositif selon l'une des revendications 1 à 5, **caractérisé en ce que** l'organe creux est l'estomac, l'œsophage ou l'utérus.

7. Dispositif selon la revendication 6, **caractérisé en ce que** la zone hémorragique (4) est la paroi interne de l'utérus d'une patiente.

8. Dispositif selon la revendication 7, **caractérisé en ce que** la zone hémorragique (4) est la région de l'insertion placentaire de la paroi interne de l'utérus d'une patiente.

## Patentansprüche

1. Hämostatische Vorrichtung (23) zur Kontrolle eines Blutflusses in einer hämorrhagischen Zone (4), die sich in einem Hohlorgan befindet, umfassend:
- eine flache elastische Platte (26), die mit zwei etwa parallelen gegenüberliegenden Flächen (27, 28) versehen ist, wobei die Platte ein leeres Innenvolumen (29) zwischen ihren zwei Flächen umfasst, und
- Anschlussmittel (30), die eingerichtet sind, um das Innenvolumen (29) an zur Vorrichtung äußere Ansaugmittel derart anzuschließen, dass ein Unterdruck in diesem Innenvolumen (29) gebildet wird,
wobei jede der Flächen (27, 28) mit Löchern (32) versehen ist, die über diese Flächen (27, 28) das Innenvolumen (29) und die Außenwelt der Platte derart in Kommunikation versetzen, dass, wenn der Unterdruck im Innenvolumen hergestellt ist, die Ansaugung gleichzeitig an zwei gegenüberliegende Wände des Hohlorgans derart kommuniziert werden kann, dass sie angenähert gehalten werden.

2. Vorrichtung nach Anspruch 1, **dadurch gekennzeichnet, dass** jede der Flächen (27, 28) der Platte zur Außenwelt der Vorrichtung (23) hin mit Reliefmotiven (35) in Höckerform versehen ist.

3. Vorrichtung nach Anspruch 2, **dadurch gekennzeichnet, dass** die Motive (35) auf den Flächen (27, 28) gleichmäßig mit einer etwa konstanten Oberflächendichte verteilt sind.

4. Vorrichtung nach einem der vorangehenden Ansprüche, **dadurch gekennzeichnet, dass** die Anschlussmittel einen Verbindungsschlauch (31) umfassen und dass sich der Schlauch (31) im Inneren des Volumens (29) über die gesamte Länge des Volumens (29) verlängert.

5. Vorrichtung nach Anspruch 4, **dadurch gekennzeichnet, dass** der Schlauch (31) über seine gesamte Länge, die sich im Inneren des Volumens verlängert, mit Paaren seitlicher Löcher (36) versehen ist, die das Innere des Schlauchs (31) mit dem Innenvolumen (29) derart in Kommunikation versetzen, dass der Unterdruck im Innenvolumen (29) schön gleichmäßig ist.

6. Vorrichtung nach einem der Ansprüche 1 bis 5, **dadurch gekennzeichnet, dass** das Hohlorgan der Magen, die Speiseröhre oder der Uterus ist.

7. Vorrichtung nach Anspruch 6, **dadurch gekennzeichnet, dass** die hämorrhagische Zone (4) die Innenwand des Uterus einer Patientin ist.

8. Vorrichtung nach Anspruch 7, **dadurch gekennzeichnet, dass** die hämorrhagische Zone (4) die Region der Plazentainsertion der Innenwand des Uterus einer Patientin ist.

## Claims

1. Haemostatic device (23) for controlling a blood flow occurring in a haemorrhagic zone (4) located in a hollow organ, comprising:
- a flat flexible plate (26) provided with two substantially parallel, opposite faces (27, 28), said plate comprising an empty internal volume (29) between its two faces, and
- connecting means (30) arranged for connecting the internal volume (29) to aspirating means external to said device, so as to create negative pressure in this internal volume (29),
each of the faces (27, 28) being provided with holes (32) configured so that the internal volume (29) and the exterior of the plate communicate via these faces (27, 28), such that when a negative pressure is created in the internal volume, the aspiration can be communicated simultaneously to two opposite walls of the hollow organ so as to hold them close together.

2. Device according to claim 1, **characterised in that** each of the faces (27, 28) is provided, towards the exterior of the device (23), with relief patterns (35) in the form of bumps.

3. Device according to claim 2, **characterised in that** the patterns (35) are evenly distributed on the faces (27, 28), with a substantially constant surface density.

4. Device according to any one of the preceding claims, **characterised in that** said connecting means comprise a connecting tube (31), and **in that** the tube (31) extends inside the volume (29), over the whole length of the volume (29).

5. Device according to claim 4, **characterised in that** the tube (31) is provided, on its entire length extending within the internal volume, with pairs of lateral holes (36) so that the interior of the tube (31) communicates with the internal volume (29), such that the negative pressure is properly uniform within the internal volume (29).

6. Device according to one of claims 1 to 5, **characterised in that** the hollow organ is the stomach, oesophagus, or uterus.

7. Device according to claim 6, **characterised in that** the haemorrhagic zone (4) is the inside wall of a patient's uterus.

8. Device according to claim 7, **characterised in that** the haemorrhagic zone (4) is the region of placental insertion of the inside wall of a patient's uterus.
